# EUROPEAN PATENT APPLICATION

(11) **EP 4 085 941 A1**
(43) Date of publication of application: **09.11.2022**
(21) Application number: 21774393.9
(22) Date of filing: 13.01.2021
(51) Int. Cl.: A61M 5/142, A61M 39/10

(54) **MEDICINAL SOLUTION ADMINISTRATION DEVICE**

(30) Priority: 23.03.2020 JP 2020050641
(71) Applicant: TERUMO Kabushiki Kaisha, Tokyo 151-0072 (JP)
(72) Inventor: UCHIYAMA Joji, Ashigarakami-gun, Kanagawa 259-0151 (JP); YAMAZAKI Tsuyoshi, Ashigarakami-gun, Kanagawa 259-0151 (JP)
(74) Representative: Casalonga
(86) International application number: PCT/JP2021/000769
(87) International publication number: WO 2021/192501

(57) **Abstract**

A medicinal solution administration device includes a device main body and a cradle. A first connection portion is formed in the device main body, and the cradle is provided with a connection port. The connection port has a second connection portion. The second connection portion has a medicinal solution intake port portion, a spring, a steel ball, and an O-ring. In a case where the device main body is mounted on the cradle, a medicinal solution is capable of flowing into the medicinal solution intake port portion by a liquid delivery port portion displacing the steel ball in the medicinal solution intake port against a biasing force of the spring. In a case where the device main body is removed from the cradle, a distal portion of the medicinal solution intake port portion is blocked by the steel ball being biased to the O-ring side by the biasing force of the spring.

## Description

### Technical Field

The present invention relates to a medicinal solution administration device for medicinal solution administration to an object such as a living body.

### Background Art

A treatment method for continuously administering a medicinal solution into a patient's body is known. For example, by a known treatment method for a diabetic patient, a small amount of insulin is continuously administered into the patient's body. Used for this treatment method is a portable medicinal solution administration device that is fixed to a patient's body or clothing. With the portable medicinal solution administration device, a medicinal solution can be administered to a patient all day long. An insulin pump for insulin administration to a patient is known as this type of medicinal solution administration device.

A syringe pump-type medicinal solution administration device that has a syringe storing a medicinal solution and a plunger driven in the syringe has been proposed as one of such portable medicinal solution administration devices. As for the syringe pump-type medicinal solution administration device, a cannula is liquid-tightly connected to a liquid delivery tube extending from the syringe, the cannula is placed by stabbing under the skin of a patient, and the medicinal solution stored in the syringe is administered into the patient's body by driving the plunger.

As for the techniques described in PTL 1 and PTL 2, the distal portion of a liquid delivery tube is provided with a connecting needle tube, a rubber partition wall is provided at a connection port having a cannula, and the liquid delivery tube and the cannula are connected by piercing the partition wall with the connecting needle tube.

### Citation List

### Patent Literature

PTL 1: International Publication No. 2006/108809
PTL 2: International Publication No. 2008/078318

### Summary of Invention

### Technical Problem

The techniques described in PTL 1 and PTL 2 have the following problems. A medicinal solution administration device such as an insulin pump includes a device main body and a cradle that can be detached from each other. The cradle is attached and fixed to a patient's skin, and the device main body is attached to and detached from the cradle. The syringe, plunger, liquid delivery tube, and connecting needle tube described above are provided in the device main body, and the connection port described above is provided in the cradle. When the device main body is mounted on the cradle, the partition wall is punctured with the connecting needle tube.

During the use of the medicinal solution administration device, the device main body needs to be removed from the cradle for some reason (e.g. patient taking a bath). After the bath, the device main body needs to be re-mounted on the cradle. As a result, attachment and detachment between the device main body and the cradle are repeated during the use of the medicinal solution administration device, and the partition wall is punctured with the connecting needle tube and the connecting needle tube is removed from the partition wall by the repetition count. This results in, for example, a wide puncture hole in the partition wall or damage to the partition wall itself, and then medicinal solution leakage may occur from the connection point between the partition wall and the connecting needle tube.

An object of the invention is to provide a medicinal solution administration device with which it is possible to improve partition wall durability pertaining to repeated attachment and detachment between a device main body and a cradle.

### Solution to Problem

A medicinal solution administration device according to the invention includes a device main body and a cradle detachable from each other. In the device main body, a liquid delivery tube extending from a medicinal solution storage portion is provided and a first connection portion where a liquid delivery hole extending from the liquid delivery tube is open is formed. The cradle is provided with a connection port connected to the liquid delivery tube via the first connection portion. The connection port has a second connection portion connectable to the first connection portion. The second connection portion has a medicinal solution intake port portion, a seal portion provided in the medicinal solution intake port portion, a plug portion provided in the medicinal solution intake port portion and movable in the medicinal solution intake port portion, and a biasing unit blocking a distal portion of the medicinal solution intake port portion by pressing the plug portion against the seal portion. In a case where the device main body is mounted on the cradle, a medicinal solution is capable of flowing into the medicinal solution intake port portion from the liquid delivery tube through the liquid delivery hole by the first connection portion displacing the plug portion in the medicinal solution intake port portion against a biasing force of the biasing unit. In a case where the device main body is removed from the cradle, the distal portion of the medicinal solution intake port portion is blocked by the plug portion being biased toward the seal portion by the biasing force of the biasing unit.

### Advantageous Effect of Invention

According to the invention, it is possible to suppress partition wall damage and medicinal solution leakage attributable to repeated attachment and detachment between a device main body and a cradle.

### Brief Description of Drawings

[Fig. 1] Fig. 1 is an exploded perspective view illustrating an overall configuration of a medicinal solution administration device according to a first embodiment of the invention.
[Fig. 2] Fig. 2 is a plan view illustrating a main part of the medicinal solution administration device illustrated in Fig. 1.
[Fig. 3] Fig. 3 is a perspective view illustrating an overall configuration of a device main body in the first embodiment of the invention.
[Fig. 4] Fig. 4 is an enlarged view of a part of the device main body illustrated in Fig. 3.
[Fig. 5] Fig. 5 is a side view of a connection port of the medicinal solution administration device according to the first embodiment of the invention.
[Fig. 6] Fig. 6 is an exploded perspective view of the connection port illustrated in Fig. 5.
[Fig. 7] Fig. 7 is an exploded cross-sectional view of the connection port illustrated in Fig. 5.
[Fig. 8] Fig. 8 is a perspective view illustrating the slide direction at a time when the device main body is attached and detached with respect to a cradle.
[Fig. 9] Fig. 9 is a cross-sectional view illustrating the state of disposition of each part before connecting a first connection portion and a second connection portion in the medicinal solution administration device according to the first embodiment of the invention.
[Fig. 10] Fig. 10 is a cross-sectional view illustrating the state of disposition of each part after connecting the first connection portion and the second connection portion in the medicinal solution administration device according to the first embodiment of the invention.
[Fig. 11] Fig. 11 is an exploded perspective view of a connection port of a medicinal solution administration device according to a second embodiment of the invention.
[Fig. 12] Fig. 12 is a cross-sectional view illustrating the state of disposition of each part before connecting a first connection portion and a second connection portion in the medicinal solution administration device according to the second embodiment of the invention.
[Fig. 13] Fig. 13 is a cross-sectional view illustrating the state of disposition of each part after connecting the first connection portion and the second connection portion in the medicinal solution administration device according to the second embodiment of the invention.
[Fig. 14] Fig. 14 is a perspective view illustrating a main part of a device main body in a third embodiment of the invention.
[Fig. 15] Fig. 15 is a perspective view of a connection port of a medicinal solution administration device according to the third embodiment of the invention.
[Fig. 16] Fig. 16 is an exploded perspective view of the connection port of the medicinal solution administration device according to the third embodiment of the invention.
[Fig. 17] Fig. 17 is a cross-sectional view illustrating the state of disposition of each part before connecting a first connection portion and a second connection portion in the medicinal solution administration device according to the third embodiment of the invention.
[Fig. 18] Fig. 18 is a cross-sectional view illustrating the state of disposition of each part after connecting the first connection portion and the second connection portion in the medicinal solution administration device according to the third embodiment of the invention.
[Fig. 19] Fig. 19 is a perspective view illustrating a modification example of the connection port of the medicinal solution administration device according to the third embodiment of the invention.

### Description of Embodiments

Hereinafter, embodiments of the invention will be described in detail with reference to the drawings. In the present specification and drawings, elements substantially identical in function or configuration are denoted by the same reference numerals without redundant description.

### [First Embodiment]

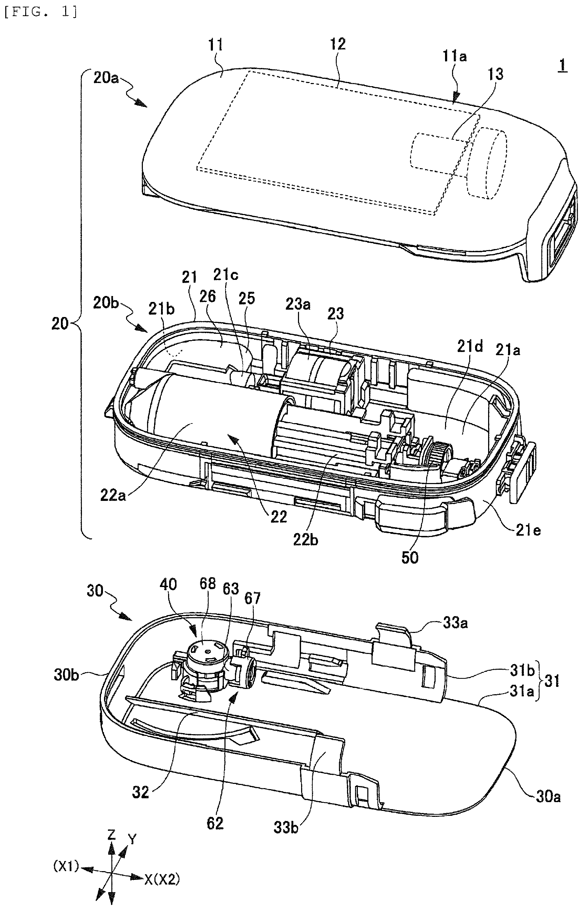

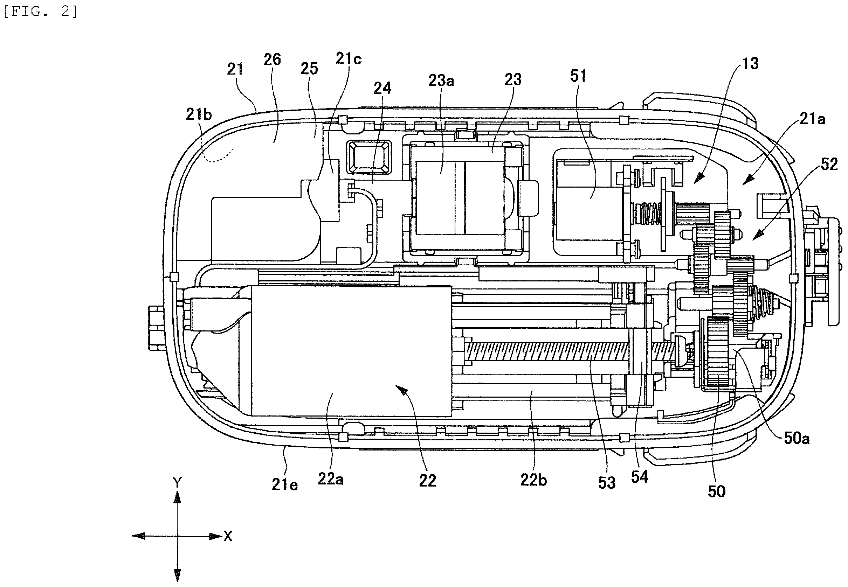

Fig. 1 is an exploded perspective view illustrating an overall configuration of a medicinal solution administration device according to a first embodiment of the invention, and Fig. 2 is a plan view illustrating a main part of the medicinal solution administration device illustrated in Fig. 1. As illustrated in Figs. 1 and 2, a medicinal solution administration device 1 includes a device main body 20 and a cradle 30. In addition, the device main body 20 includes a first main body portion 20a and a second main body portion 20b. The first main body portion 20a is a reuse portion. The second main body portion 20b is a disposal portion. The first main body portion 20a and the second main body portion 20b can be separated from each other and can be detached from each other. The device main body 20 functions by operably connecting the first main body portion 20a and the second main body portion 20b.

The medicinal solution administration device 1 is a device for continuously administering a medicinal solution into the body of a patient. Examples of the medicinal solution administered using the medicinal solution administration device 1 include insulin, an analgesic, an anticancer therapeutic agent, a human immunodeficiency virus (HIV) therapeutic agent, an iron chelating agent, and a pulmonary hypertension therapeutic agent. In the present embodiment, a portable insulin pump for administering insulin into the body of a patient is assumed as an example of the medicinal solution administration device 1. The medicinal solution administration device 1 that is the insulin pump is used by being attached to the surface of a living body. The living body surface is typically a patient's skin surface. In addition, the position where the medicinal solution administration device 1 is attached is, for example, a patient's abdomen.

The X, Y, and Z directions in the following description are defined as follows in order to clarify the shape and positional relationship of each part of the medicinal solution administration device 1. First, in a case where the device main body 20 is mounted on the cradle 30, the device main body 20 is slid from a first end 30a toward a second end 30b in the longitudinal direction of the cradle 30. The X direction is parallel to the direction in which the device main body 20 is slid. In addition, the X direction is parallel to the longitudinal direction of the cradle 30. The Z direction is parallel to the direction in which the device main body 20 and the cradle 30 overlap with the device main body 20 mounted on the cradle 30. In addition, the Z direction is orthogonal to a living body surface when the medicinal solution administration device 1 is attached to the living body surface and the medicinal solution is administered. In the present specification, the Z direction is divided into upward and downward directions. The upward direction is the direction in which the first main body portion 20a is positioned when viewed from the cradle 30 with the device main body 20 and the cradle 30 operably connected to each other. The downward direction is the direction in which the cradle 30 is positioned when viewed from the first main body portion 20a with the device main body 20 and the cradle 30 operably connected to each other. In addition, the downward direction is the direction in which the medicinal solution administration device 1 that is mounted on a living body surface faces the living body surface. As for the X direction, the direction from the first end 30a to the second end 30b is an X1 direction and the direction from the second end 30b to the first end 30a is an X2 direction in a top view of the medicinal solution administration device 1. The Y direction is orthogonal to both the X direction and the Z direction. The Y direction is parallel to the lateral direction of the cradle 30.

### (First Main Body Portion 20a)

The first main body portion 20a has a lid body 11, a circuit board 12, and a liquid delivery drive unit 13. The first main body portion 20a is a part where the electronic control functions of the device main body 20 are integrated. The first main body portion 20a is configured by accommodating electronic control function components such as the circuit board 12 and the liquid delivery drive unit 13 inside the lid body 11. The liquid delivery drive unit 13 is configured by a motor, a gear, and so on. The motor is a drive source for driving a plunger 22b. The plunger 22b is accommodated in the second main body portion 20b, which will be described later.

The lid body 11 is configured to be engageable with a housing 21 of the second main body portion 20b. An upper surface 11a of the lid body 11 forms the top surface of the medicinal solution administration device 1. The electronic control function components such as the circuit board 12 and the liquid delivery drive unit 13 are attached to the lower surface side of the lid body 11.

### (Second Main Body Portion 20b)

The second main body portion 20b includes the housing 21, a medicinal solution storage portion 22, and a battery box 23. The housing 21 is formed in a substantially rectangular shape in a plan view. In addition, the housing 21 is a rectangular parallelepiped having an upper opening with each corner portion chamfered in a round shape. The opening portion of the housing 21 is configured to be closed from above by the lid body 11 of the first main body portion 20a.

The housing 21 has a first storage portion 21a, a second storage portion 21b, a first connection portion 21c, a bottom plate portion 21d, and a side plate portion 21e. The first storage portion 21a is a space surrounded by the bottom plate portion 21d and the side plate portion 21e. The first storage portion 21a is formed on the upper surface side of the housing 21. In addition, the first storage portion 21a is formed so as to be concave in a substantially U shape. The medicinal solution storage portion 22, a battery 23a, and so on are stored in the first storage portion 21a. In addition, when the lid body 11 and the housing 21 are engaged with each other, the liquid delivery drive unit 13 attached to the inside of the lid body 11 is accommodated in the first storage portion 21a. The volume of the first storage portion 21a is larger than the volume of the second storage portion 21b.

The second storage portion 21b is formed on the lower surface side of the housing 21. The second storage portion 21b is formed in a substantially U shape and concave upward. A connection port 40 of the cradle 30 and so on are stored in the second storage portion 21b. The first storage portion 21a and the second storage portion 21b are partitioned by a first partition wall 25. A second partition wall 26 is provided in the vicinity of the first partition wall 25. The first partition wall 25 and the second partition wall 26 are continuously provided. The first partition wall 25 extends in the substantially Z direction, and the second partition wall 26 extends in the substantially X direction.

### (First Connection Portion 21c)

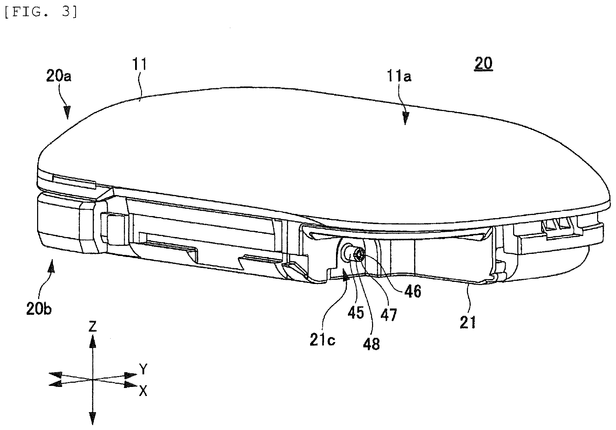

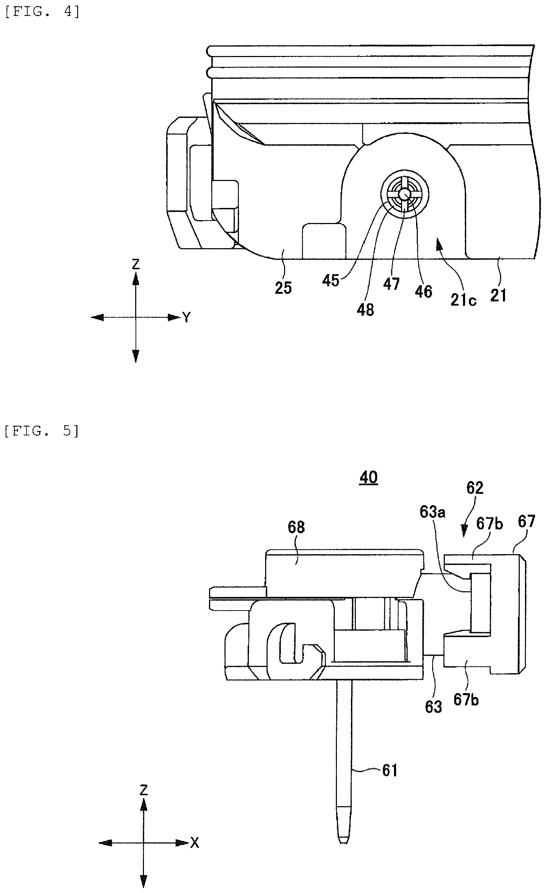

The first connection portion 21c is a part connected to a second connection portion 62 of the connection port 40. The first connection portion 21c is provided on the surface of the first partition wall 25 on the X1 direction side. As illustrated in Figs. 3 and 4, the first connection portion 21c is formed in an inverted U shape so as to be concave in the X2 direction when viewed from the X direction. The first connection portion 21c is provided with a liquid delivery port portion 45. The liquid delivery port portion 45 is formed in a substantially circular shape when viewed from the X direction. In addition, the liquid delivery port portion 45 is a rigid tube formed in a convex shape so as to protrude in the X1 direction from the first partition wall 25 of the housing 21 and the end portion of the liquid delivery port portion 45 in the direction in which the liquid delivery port portion 45 protrudes is the distal portion of the liquid delivery port portion 45.

The liquid delivery port portion 45 has a liquid delivery hole 46 and an outer peripheral surface surrounding the liquid delivery hole 46. The liquid delivery hole 46 is formed so as to penetrate the liquid delivery port portion 45 along the central axis of the liquid delivery port portion 45. The liquid delivery hole 46 is open at the distal position of the liquid delivery port portion 45 in the first connection portion 21c. In addition, four grooves 47 are formed in the distal portion of the liquid delivery port portion 45. The four grooves 47 are formed radially (that is, in a cross shape) around the liquid delivery hole 46. Further, a first tapered guide 48 is formed on the distal portion of the liquid delivery port portion 45. The first tapered guide 48 is formed in a rounded and inclined shape with respect to the central axis of the liquid delivery port portion 45 (that is, the opening center of the liquid delivery hole 46).

Returning to Figs. 1 and 2, the bottom plate portion 21d is a plate portion that partitions the housing 21 in the Z direction. The bottom plate portion 21d is disposed so as to face the cradle 30 in a case where the device main body 20 is mounted on the cradle 30. The side plate portion 21e is a plate portion that surrounds the outer peripheral portion of the housing 21. The side plate portion 21e is formed so as to stand vertically from the outer peripheral portion of the bottom plate portion 21d.

### (Medicinal Solution Storage Portion 22)

The medicinal solution storage portion 22 is configured by a syringe including an outer tube 22a in which a medicinal solution to be administered to a patient is stored and the plunger 22b inserted into the outer tube 22a. The medicinal solution storage portion 22 is stored in the first storage portion 21a of the housing 21. A medicinal solution discharge port (not illustrated) is provided at the distal end of the outer tube 22a, and one end of a liquid delivery tube 24 is connected to the discharge port. The other end of the liquid delivery tube 24 is connected to the liquid delivery hole 46 (see Figs. 3 and 4) of the liquid delivery port portion 45 on the first storage portion 21a side of the housing 21. In other words, the lumen of the liquid delivery tube 24 extends to the opening of the liquid delivery hole 46. The medicinal solution storage portion 22 is not limited to the syringe and may be any that is capable of storing a medicinal solution, examples of which include a soft bag.

A gear 50 is provided near an end portion of the plunger 22b. The gear 50 rotates by receiving the drive force of the liquid delivery drive unit 13. Fig. 2 illustrates a state where the liquid delivery drive unit 13 is disposed in the first storage portion 21a of the housing 21. The liquid delivery drive unit 13 includes a motor 51 and a gear train 52. The gear train 52 is configured by a plurality of stages of gears, and the gear 50 meshes with the gear of the final stage. In addition, a feed screw 53 meshes with an end portion of a rotating shaft 50a of the gear 50. In addition, a nut 54 meshes with the screw shaft of the feed screw 53. The nut 54 is provided so as to be movable in the X direction. The plunger 22b is pushed by the movement of the nut 54.

In the liquid delivery drive unit 13 and the medicinal solution storage portion 22 configured as described above, the feed screw 53 rotates in accordance with the rotation of the gear 50 when the gear train 52 and the gear 50 are rotated by the drive of the motor 51. In addition, when the feed screw 53 rotates, the nut 54 moves integrally with the plunger 22b to one side in the X direction, that is, the outer tube 22a side. As a result, the plunger 22b is pushed into the outer tube 22a and the medicinal solution is sent out from the outer tube 22a to the liquid delivery tube 24 in accordance with the pushing amount of the plunger 22b.

The battery box 23 is a box where the battery 23a is stored. The battery 23a serves as a power source for driving the liquid delivery drive unit 13 or the like. The battery 23a is connected to an electrode (not illustrated) on the circuit board 12. The battery box 23 and the battery 23a may be disposed inside the lid body 11 as elements of the first main body portion 20a.

### (Cradle 30)

The cradle 30 includes a bottom case 31 and the connection port 40. The bottom case 31 has a placement surface portion 31a and a side wall portion 31b. The connection port 40 is attached to the bottom case 31. The lower surface of the bottom case 31 is provided with an adhesive portion (not illustrated). The adhesive portion is a part for attaching the cradle 30 to a patient's skin. The placement surface portion 31a is formed in a substantially rectangular shape in a plan view. The placement surface portion 31a has four corner portions, and each corner portion is rounded. In a case where the device main body 20 is mounted on the cradle 30, the housing 21 of the device main body 20 is placed on the placement surface portion 31a. The side wall portion 31b is formed so as to stand from three sides of the outer periphery of the placement surface portion 31a, and the side wall portion 31b is not formed on the remaining side of the placement surface portion 31a. In other words, one side of the outer periphery of the placement surface portion 31a is open. The reason for opening one side of the placement surface portion 31a is to mount the device main body 20 on the cradle 30 in a sliding manner.

In the bottom case 31, a rail portion 32 of a ridge and a pair of hold portions 33a and 33b are integrally formed. The rail portion 32 is formed on the placement surface portion 31a of the bottom case 31 in parallel with the X direction. The pair of hold portions 33a and 33b are formed so as to face each other in the Y direction and are curved and stand up from the upper edge of the side wall portion 31b. The curved shape of the hold portions 33a and 33b corresponds to the shape of the lid body 11 of the first main body portion 20a. The rail portion 32 guides the device main body 20 when the device main body 20 is mounted on the cradle 30, and the pair of hold portions 33a and 33b correct the attachment posture of the device main body 20 by holding the lid body 11 of the device main body 20 such that the lid body 11 can be held from both sides.

### (Connection Port 40)

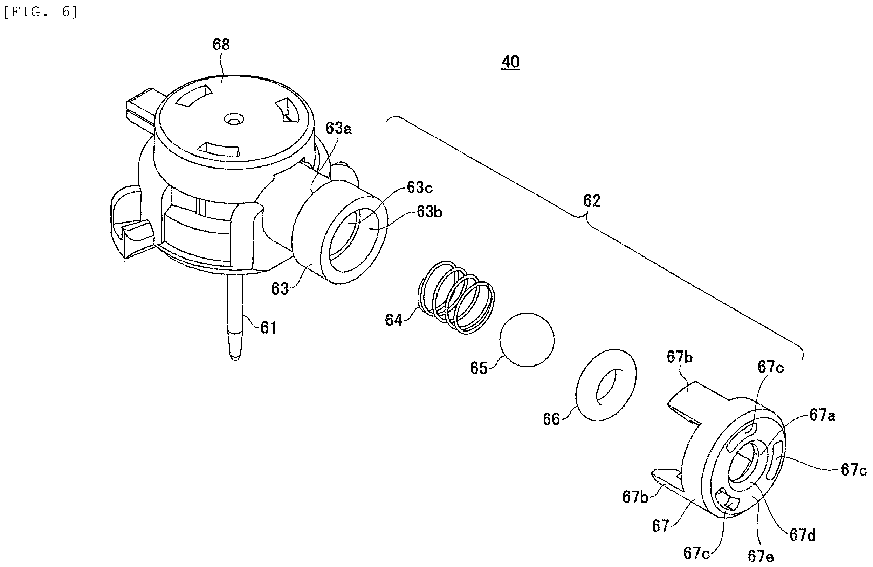

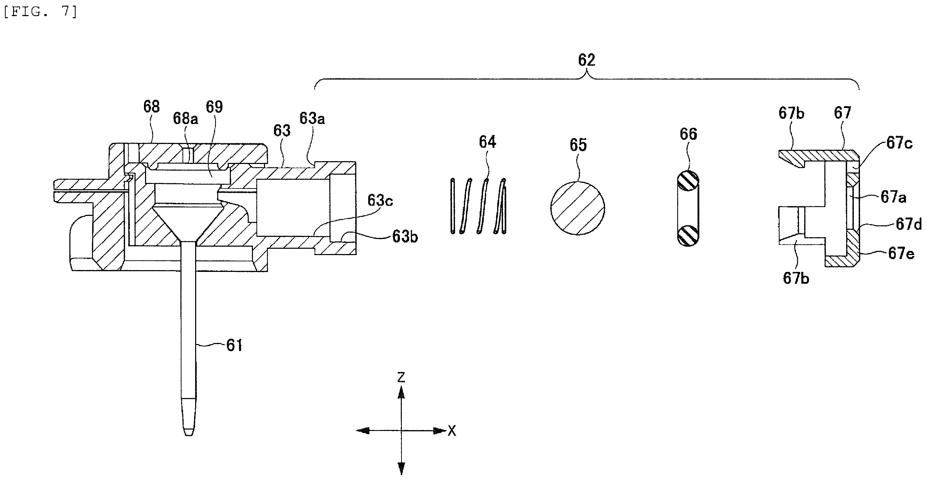

Fig. 5 is a side view of the connection port 40 of the medicinal solution administration device 1 according to the first embodiment of the invention. Fig. 6 is an exploded perspective view of the connection port 40 illustrated in Fig. 5, and Fig. 7 is an exploded sectional view of the connection port 40 illustrated in Fig. 5. As illustrated in Figs. 5 to 7, the connection port 40 includes a housing 68, a cannula 61, the second connection portion 62, and a rubber stopper 69. The connection port 40 is connected to the liquid delivery tube 24 via the first connection portion 21c. The cannula 61 is a tubular member with which a living body is punctured and which introduces a medicinal solution such as insulin into the living body in the puncture state. The cannula 61 is made of, for example, a resin material. The resin material that forms the cannula 61 is, for example, polyurethane, nylon, ethylene-tetrafluoroethylene copolymer (ETFE), or the like. The cannula 61 is inserted into the living body using a puncture device (not illustrated). In a ready-to-use state, the puncture device is attached to the cradle 30 together with the connection port 40. At that time, an inner needle (not illustrated) is passed through the cannula 61. The cannula 61 is inserted into the living body together with the inner needle. The inner needle is accommodated in the puncture device through the rubber stopper 69 above the proximal side of the cannula 61 and an opening portion 68a in the upper portion of the housing 68, and only the cannula 61 is inserted into the living body. After the cannula 61 is inserted into the living body, the puncture device is removed from the cradle 30 and discarded. The rubber stopper 69 may be any insofar as an inner needle can be inserted through the rubber stopper 69 and the rubber stopper 69 itself is capable of self-sealing. An elastic member such as an elastomer and natural rubber can be used as the rubber stopper 69. Given the risk of infection, it is preferable that the inner needle and the hub that holds the inner needle are disposable.

The second connection portion 62 includes a medicinal solution intake port portion 63, a spring 64, a steel ball 65, an O-ring 66, and a cap portion 67. The medicinal solution intake port portion 63 is formed in a cylindrical shape and protrudes in the X2 direction. A stepped portion 63a is formed on the outer peripheral side of the medicinal solution intake port portion 63. By the stepped portion 63a being formed, the outer diameter of the distal side of the medicinal solution intake port portion 63 is larger than the outer diameter of the proximal side of the medicinal solution intake port portion 63. A first hole portion 63b and a second hole portion 63c are formed on the inner peripheral side of the medicinal solution intake port portion 63. The diameter of the first hole portion 63b is set to be larger than the diameter of the second hole portion 63c. The second hole portion 63c communicates with the cannula 61 in the housing 68. In the present specification, the term of "communication" means spatial connection. The medicinal solution intake port portion 63 is formed integrally with the housing 68. The housing 68 configures the main body part of the connection port 40. The cannula 61 described above is attached toward the lower side of the housing 68.

The spring 64 is provided in the medicinal solution intake port portion 63. In the present embodiment, the spring 64 is configured by a coil spring as an example. The spring 64 is made of spring steel such as stainless steel. The spring 64 is provided as an example of a biasing unit that seals the distal portion of the medicinal solution intake port portion 63 by pressing the steel ball 65 against the O-ring 66. The biasing unit is configured by an elastic member such as the spring 64 and applies an elastic force in the X direction (more specifically, toward the X2 direction). One end of the spring 64 comes into contact with the steel ball 65, and the other end of the spring 64 is locked to the inner surface of the medicinal solution intake port portion 63. However, the elastic member is not limited to the spring 64 and may be configured by, for example, a tubular or columnar member that has rubbery elasticity. In a case where the spring 64 is used as the elastic member, a satisfactory biasing force (elastic force) can be maintained for a long time.

The steel ball 65 is provided as an example of a plug portion that blocks a liquid introduction hole 67a of the cap portion 67. The steel ball 65 is provided in the medicinal solution intake port portion 63 together with the spring 64. In addition, the steel ball 65 is provided so as to be movable in the central axis direction of the medicinal solution intake port portion 63, that is, the X direction. The steel ball 65 receives the biasing force of the spring 64 and inscribes with the O-ring 66. As a result, the steel ball 65 blocks the flow path leading to the liquid introduction hole 67a. The diameter of the steel ball 65 is set to be smaller than the diameter of the second hole portion 63c of the medicinal solution intake port portion 63. The steel ball 65 is formed of a metal material for medical use, and the surface of the steel ball 65 may be treated if necessary. Although the steel ball 65 here moves in the central axis direction of the medicinal solution intake port portion 63 (that is, the X direction), the direction in which the plug portion moves is not limited thereto. The direction in which the plug portion moves and the direction of connection between the connection port 40 and the liquid delivery tube 21 may be the same.

The O-ring 66 configures a seal portion. The O-ring 66 is provided in the medicinal solution intake port portion 63 together with the spring 64 and the steel ball 65 described above. Specifically, the O-ring 66 is disposed in the first hole portion 63b of the medicinal solution intake port portion 63. The outer peripheral surface of the O-ring 66 is in close contact with the inner peripheral surface of the first hole portion 63b. In addition, in a case where the cap portion 67 is attached to the medicinal solution intake port portion 63, the O-ring 66 is disposed between the steel ball 65 and the cap portion 67 and is disposed in the peripheral edge portion of the liquid introduction hole 67a of the cap portion 67. In this case, the spring 64 presses the steel ball 65 against the O-ring 66 and biases the steel ball 65 toward the liquid introduction hole 67a of the cap portion 67. The spring 64, the O-ring 66, and the steel ball 65 configure a non-puncture-type sealing mechanism in the second connection portion 62. In the present specification, the term of "non-puncture-type" means "not a type in which a rubber member is punctured with a needle member". An elastic member such as an elastomer and natural rubber may be used for the O-ring 66.

The cap portion 67 is attached to the distal portion of the medicinal solution intake port portion 63. The distal portion of the medicinal solution intake port portion 63 is the end portion on the side facing the first connection portion 21c (see Fig. 3) when the device main body 20 is mounted on the cradle 30. The cap portion 67 has the liquid introduction hole 67a, a plurality of locking claws 67b, and a plurality of punching holes 67c. The O-ring 66, the spring 64, and the steel ball 65 are held in the lumen of the medicinal solution intake port portion 63 by the cap portion 67. In addition, the cap portion 67 is provided with a second tapered guide 67d.

The liquid introduction hole 67a is an opening formed at a front plate part 67e of the cap portion 67 and formed in a circular shape when the cap portion 67 is viewed from the X direction. The liquid introduction hole 67a is a through hole leading to the inner portion of the medicinal solution intake port portion 63 in a case where the cap portion 67 is attached to the medicinal solution intake port portion 63. The liquid introduction hole 67a is provided at the center of the cap portion 67 when viewed from the X direction. The front plate part 67e of the cap portion 67 covers at least a part of the O-ring 66 configuring the seal portion when viewed from the insertion direction of the medicinal solution intake port portion 63. As a result, it is possible to prevent the O-ring 66 from being damaged by the medicinal solution intake port portion 63 accidentally hitting the O-ring 66 when the medicinal solution intake port portion 63 enters the liquid introduction hole 67a.

The locking claw 67b is a part locked to the stepped portion 63a of the medicinal solution intake port portion 63 in a case where the cap portion 67 is attached to the medicinal solution intake port portion 63. The term of "locking" means to hook and stop. Three locking claws 67b are provided at a uniform angular pitch (120-degree pitch) in the circumferential direction of the cap portion 67. The punching hole 67c is provided so as to penetrate the front plate part 67e of the cap portion 67 in the X direction. Three punching holes 67c are provided at a uniform angular pitch in the circumferential direction of the cap portion 67. In addition, the punching hole 67c is formed in a long hole shape in which the circumferential direction of the cap portion 67 is the long axis direction. In the present embodiment, the cap portion 67 may or may not have the punching hole 67c. In addition, it is preferable to adopt a configuration without the punching hole 67c in a case where the distal portion of the medicinal solution intake port portion 63 is blocked by a plug portion and the presence of the punching hole 67c hinders the blockage. The necessity of the punching hole 67c is similarly applied to a second embodiment, which will be described later.

The second tapered guide 67d is formed so as to correspond to the first tapered guide 48 (see Figs. 3 and 4) described above. The second tapered guide 67d is formed on the inner peripheral edge of the liquid introduction hole 67a. In addition, the second tapered guide 67d is formed by a surface obliquely inclined with respect to the central axis of the cap portion 67, that is, a slope. The first tapered guide 48 and the second tapered guide 67d engage with each other to guide the distal portion of the liquid delivery port portion 45 to the liquid introduction hole 67a of the cap portion 67.

### <Assembly of Connection Port 40>

In assembling the connection port 40 configured as described above, the spring 64 and the steel ball 65 are inserted in order into the second hole portion 63c of the medicinal solution intake port portion 63 and, with the O-ring 66 inserted in the first hole portion 63b of the medicinal solution intake port portion 63, the cap portion 67 is attached to the distal portion of the medicinal solution intake port portion 63. By locking the locking claw 67b of the cap portion 67 to the stepped portion 63a of the medicinal solution intake port portion 63 at this time, the cap portion 67 is prevented from coming off from the medicinal solution intake port portion 63. In addition, in the medicinal solution intake port portion 63, a gap is formed between the spherical surface of the steel ball 65 and the inner peripheral surface of the second hole portion 63c. This is because the diameter of the steel ball 65 is set to be smaller than the diameter of the second hole portion 63c of the medicinal solution intake port portion 63. In addition, in the medicinal solution intake port portion 63, the steel ball 65 is pressed against the liquid introduction hole 67a side of the cap portion 67, that is, the O-ring 66 by the biasing force of the spring 64. As a result, the liquid introduction hole 67a of the cap portion 67 is sealed by the steel ball 65. In addition, a part of the outer surface of the biased steel ball 65 comes into close contact with the O-ring 66 and the O-ring 66 comes into close contact with the inner peripheral surface of the first hole portion 63b. As a result, the distal portion of the medicinal solution intake port portion 63 is liquid-tightly sealed. The term of "liquid-tight" means that no liquid leaks out.

Next, a case where the device main body 20 is mounted on the cradle 30 and a case where the device main body 20 is removed from the cradle 30 in the medicinal solution administration device according to the first embodiment of the invention will be described.

### <Case Where Device Main Body 20 Is Mounted on Cradle 30>

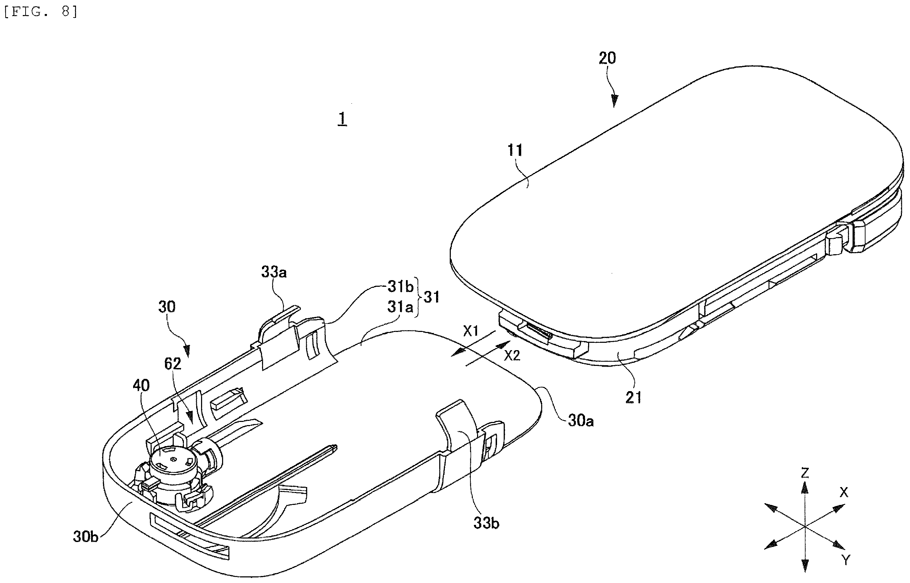

In a case where the device main body 20 is mounted on the cradle 30 in using the medicinal solution administration device 1, the device main body 20 is slid in the X1 direction with respect to the cradle 30 as illustrated in Fig. 8. At this time, the bottom surface of the housing 21 is placed on one end portion of the placement surface portion 31a of the cradle 30 and the lid body 11 is passed between the pair of hold portions 33a and 33b. As a result, the device main body 20 slides from the first end 30a toward the second end 30b of the cradle 30 and the first connection portion 21c and the second connection portion 62 are connected as a result of this sliding. The following is a detailed description.

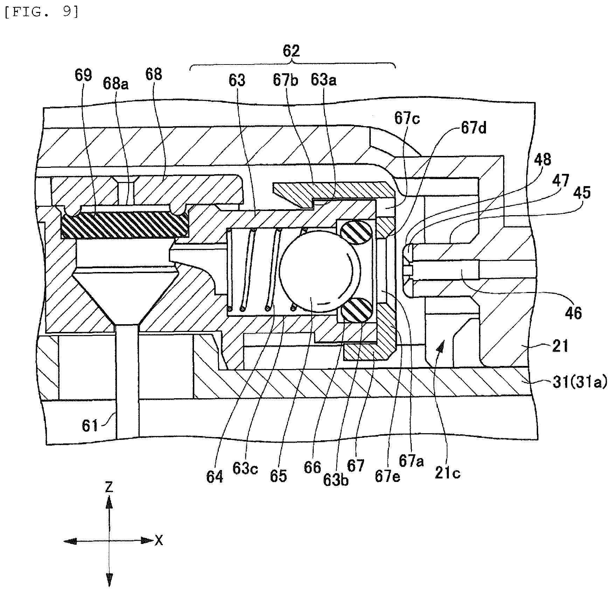

When the device main body 20 is slid in the X1 direction with respect to the cradle 30, first, the distal portion of the liquid delivery port portion 45 is disposed in the vicinity of the cap portion 67 as illustrated in Fig. 9. Next, the distal portion of the liquid delivery port portion 45 is inserted into the liquid introduction hole 67a of the cap portion 67. At this time, the distal portion of the liquid delivery port portion 45 is guided to the liquid introduction hole 67a of the cap portion 67 by the first tapered guide 48 and the second tapered guide 67d. Next, the distal portion of the liquid delivery port portion 45 comes into contact with the surface of the steel ball 65 through the liquid introduction hole 67a of the cap portion 67 and the hole of the O-ring 66. At this time, a space is formed between the spherical surface of the steel ball 65 and the distal surface of the liquid delivery port portion 45 by the groove 47 formed in the distal portion of the liquid delivery port portion 45. Accordingly, a gap corresponding to the recess dimension of the groove 47 is formed between the groove 47 of the liquid delivery port portion 45 and the spherical surface of the steel ball 65.

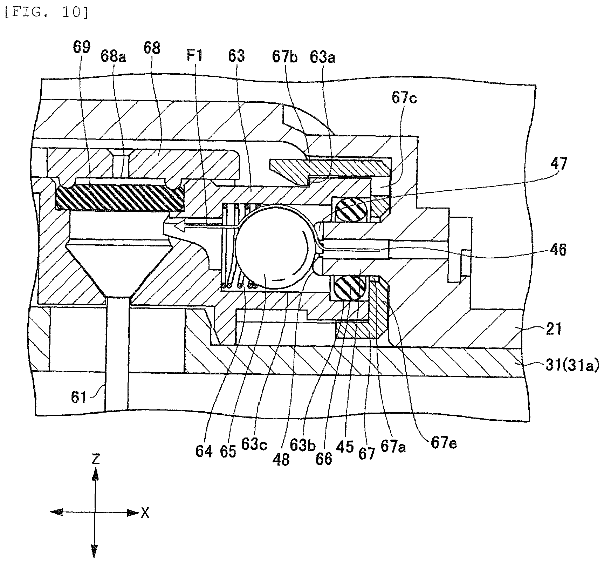

Next, the liquid delivery port portion 45 pushes the steel ball 65 into the back side of the medicinal solution intake port portion 63 against the biasing force of the spring 64. As a result of the operation of attaching the device main body 20 to the cradle 30, the steel ball 65 is displaced from the initial position to the X1 direction side in the medicinal solution intake port portion 63. In other words, the steel ball 65 moves away from the O-ring 66 and moves to the back side of the medicinal solution intake port portion 63. Then, with the device main body 20 completely mounted on the cradle 30, the liquid delivery port portion 45 is in the cap portion 67 as illustrated in Fig. 10. In this state, a medicinal solution flow path is formed by the plurality of grooves 47 at the contact part between the distal portion of the liquid delivery port portion 45 and the steel ball 65. Accordingly, the liquid delivery hole 46 of the liquid delivery port portion 45 communicates with the first hole portion 63b and the second hole portion 63c of the medicinal solution intake port portion 63 through the grooves 47. In addition, in this state, liquid-tight sealing is provided between the outer peripheral surface of the liquid delivery port portion 45 and the O-ring 66.

As a result, the first connection portion 21c and the second connection portion 62 are connected and a medicinal solution flow path F1 as indicated by an arrow is formed at the connection part. This medicinal solution flow path F1 is a flow path connected to the cannula 61 in the housing 68. Accordingly, the liquid delivery tube 24 connected to the medicinal solution storage portion 22 and the cannula 61 piercing a living body are connected by the first connection portion 21c and the second connection portion 62. Accordingly, by sending out the medicinal solution stored in the outer tube 22a of the medicinal solution storage portion 22 to the liquid delivery tube 24 with the plunger 22b, the medicinal solution is capable of flowing into the medicinal solution intake port portion 63 through the liquid delivery hole 46 and the groove 47 of the liquid delivery port portion 45. Then, the medicinal solution that has flowed into the medicinal solution intake port portion 63 is capable of flowing into the cannula 61 through the internal space of the housing 68 and can be introduced into a living body from the distal end of the cannula 61.

### <Case Where Device Main Body 20 Is Removed from Cradle 30>

In a case where the device main body 20 is removed from the cradle 30 during the use of the medicinal solution administration device 1, the device main body 20 is slid in the X2 direction (see Fig. 8), which is opposite to the mounting direction. As a result, the device main body 20 slides from the second end 30b toward the first end 30a of the cradle 30 and the first connection portion 21c and the second connection portion 62 are disconnected as a result of this sliding. The following is a detailed description.

When the device main body 20 is slid in the X2 direction with respect to the cradle 30, first, the liquid delivery port portion 45 moves in the X2 direction together with the device main body 20 and the steel ball 65 also moves in the X2 direction as the liquid delivery port portion 45 moves. At this time, the steel ball 65 is biased toward the O-ring 66 by the biasing force of the spring 64 and moves in the X2 direction. Next, the steel ball 65 comes into contact with the O-ring 66 by the liquid delivery port portion 45 moving further. As a result, the steel ball 65 is pressed against the O-ring 66 by the biasing force of the spring 64, and thus the distal portion of the medicinal solution intake port portion 63 is self-sealed by the steel ball 65. As a result, the housing 68 returns to the liquid-tightly sealed state. Next, the distal portion of the liquid delivery port portion 45 escapes to the outside of the cap portion 67 through the liquid introduction hole 67a of the cap portion 67. As a result, the first connection portion 21c and the second connection portion 62 are disconnected.

### <Effect of First Embodiment>

As described above, in the medicinal solution administration device 1 according to the first embodiment of the invention, the liquid delivery port portion 45 provided in the first connection portion 21c displaces the steel ball 65 in the medicinal solution intake port portion 63 against the biasing force of the spring 64 in a case where the device main body 20 is mounted on the cradle 30. As a result, a medicinal solution is capable of flowing into the medicinal solution intake port portion 63 from the liquid delivery tube 24 through the liquid delivery hole 46. In addition, by removing the device main body 20 from the cradle 30, the steel ball 65 is biased toward the O-ring 66 by the biasing force of the spring 64. As a result, the distal portion of the medicinal solution intake port portion 63 can be self-sealed. As a result, the housing 68 returns to the liquid-tightly sealed state. As a result, the device main body 20 and the cradle 30 can be fluidly connected repeatedly and contamination in the medicinal solution intake port 63 can be prevented. In addition, it is possible to suppress diaphragm damage and coring attributable to repeated attachment and detachment between the device main body 20 and the cradle 30. Accordingly, it is possible to suppress medicinal solution leakage attributable to repeated attachment and detachment between the device main body 20 and the cradle 30.

In addition, in the first embodiment of the invention, the cap portion 67 is attached to the distal portion of the medicinal solution intake port portion 63 and the liquid introduction hole 67a is formed in the cap portion 67. Accordingly, the cap portion 67 restricts the X-direction movement of the O-ring 66 even in a case where the steel ball 65 is strongly pressed against the O-ring 66 by the biasing force of the spring 64. In other words, the cap portion 67 fulfills the function of preventing the O-ring 66 from coming off and protects the O-ring 66. Accordingly, the O-ring 66 can be reliably held in the first hole portion 63b of the medicinal solution intake port portion 63. In addition, the distal portion of the medicinal solution intake port portion 63 can be blocked with high liquid tightness. In addition, since a plug portion is configured by using the steel ball 65, high liquid tightness can be maintained for a long time. In addition, with the device main body 20 completely mounted on the cradle 30, liquid-tight sealing is provided between the outer peripheral surface of the liquid delivery port portion 45 and the O-ring 66 in the medicinal solution intake port portion 63. As a result, even in the event of bending between the cradle 30 and the device main body 20 attributable to a body movement or the like, liquid leakage is unlikely to occur and medicinal solution delivery from the distal portion of the liquid delivery port portion 45 can be reliably ensured.

The plurality of grooves 47 are formed in the distal portion of the liquid delivery port portion 45 in the first embodiment, and yet the invention is not limited thereto. In alternative configurations, one, two, three, or five or more grooves 47 may be formed in the distal portion of the liquid delivery port portion 45. In a case where the plurality of grooves 47 are formed in the distal portion of the liquid delivery port portion 45, the flow path resistance of the medicinal solution flow path at the contact part between the distal portion of the liquid delivery port portion 45 and the steel ball 65 is smaller than in a case where only one groove 47 is formed. Accordingly, a medicinal solution can be smoothly delivered from the liquid delivery port portion 45 to the medicinal solution intake port portion 63. In addition, in a case where the plurality of grooves 47 are formed in the distal portion of the liquid delivery port portion 45, the plurality of grooves 47 are radially formed around the liquid delivery hole 46 of the liquid delivery port portion 45. As a result, a medicinal solution can be more smoothly delivered from the liquid delivery port portion 45 to the medicinal solution intake port portion 63 and the medicinal solution can be more uniformly delivered in the circumferential direction of the medicinal solution intake port portion 63.

In addition, in the first embodiment, the O-ring 66 as a seal portion and the medicinal solution intake port portion 63 are configured as separate components. The invention is not limited thereto, and the seal portion and the medicinal solution intake port portion 63 may be an integrated structure. Specifically, the seal portion and the medicinal solution intake port portion 63 may be configured by a two-color molded product. By the seal portion and the medicinal solution intake port portion 63 being an integrated structure, the number of components of the connection port 40 can be reduced. In addition, by the seal portion and the medicinal solution intake port portion 63 being configured by a two-color molded product, it is possible to save the trouble of attaching the seal portion to the medicinal solution intake port portion 63. Accordingly, the assembly man-hours of the connection port 40 can be reduced.

Further, the spring 64 as a biasing unit and the steel ball 65 as a plug portion are configured as separate components in the first embodiment, and yet the invention is not limited thereto. The spring 64 and the steel ball 65 may be an integrated structure. Joining the spring 64 and the steel ball 65 by welding or the like is conceivable as an example of how to integrate the spring 64 and the steel ball 65. By integrating the spring 64 and the steel ball 65, the number of components inserted into the medicinal solution intake port portion 63 is reduced, and thus the assembly man-hours of the connection port 40 can be reduced.

In the configuration of the first embodiment, the O-ring 66 is used as a seal portion and the distal portion of the medicinal solution intake port portion 63 is blocked by pressing the steel ball 65 against the O-ring 66. The invention is not limited thereto, and the cap portion 67 can be configured as a seal portion. Specifically, the distal portion of the medicinal solution intake port portion 63 may be blocked by the cap portion 67 being made of an elastic material such as rubber and the steel ball 65 being pressed against the liquid introduction hole 67a of the cap portion 67. In this configuration, the O-ring 66 is unnecessary, and thus the number of components of the connection port 40 can be reduced and the assembly man-hours of the connection port 40 can be reduced.

In addition, although the plug portion is configured by the steel ball 65 in the first embodiment, the invention is not limited thereto. For example, the plug portion may be configured by a sphere made of an elastic material such as rubber. In a case where this configuration is adopted, the O-ring 66 may be configured as a seal portion or the cap portion 67 may be configured as a seal portion.

### [Second Embodiment]

The second embodiment of the invention will be described below. The medicinal solution administration device 1 according to the second embodiment of the invention is different from the device configuration of the first embodiment described above in terms of the configuration of the connection port 40, the second connection portion in particular. The following is a detailed description.

### (Connection Port 40)

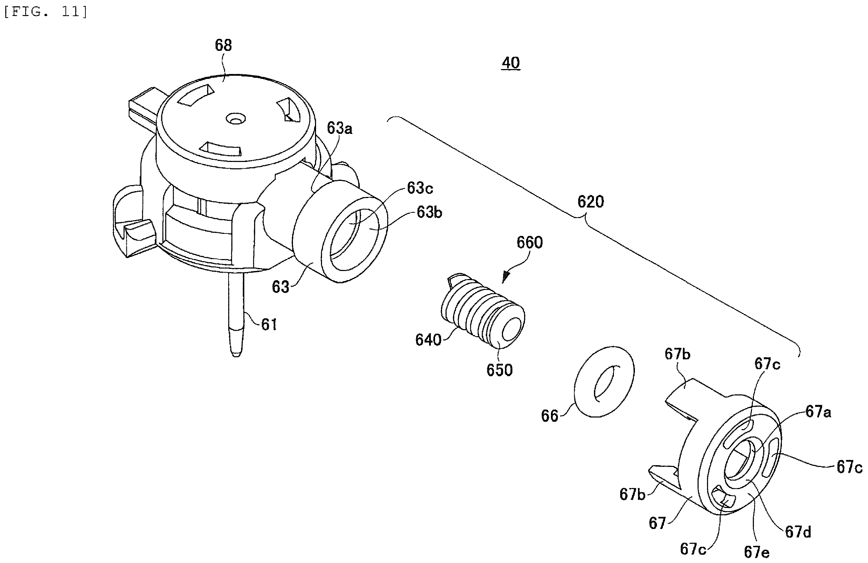

Fig. 11 is an exploded perspective view of the connection port 40 of the medicinal solution administration device 1 according to the second embodiment of the invention. As illustrated in Fig. 11, the connection port 40 includes the cannula 61, the housing 68, the rubber stopper 69, and a second connection portion 620. The configurations of the cannula 61, the housing 68, the rubber stopper 69, the puncture device (not illustrated), and the inner needle (not illustrated) are the same as those of the first embodiment. The second connection portion 620 includes the medicinal solution intake port portion 63, the O-ring 66, and the cap portion 67, the configurations of which are the same as those of the first embodiment.

In the second embodiment of the invention, a plug portion 650 and a biasing unit 640 are an integrated structure. Specifically, the plug portion 650 and the biasing unit 640 are configured by an integrally molded resin product 660. Examples of the resin configuring the integrally molded product 660 include general-purpose plastics such as polyacetal resin and ABS resin and various engineering plastics. The plug portion 650 is formed in a disk shape. The biasing unit 640 is formed in a spiral shape and has an appropriate springiness.

### <Assembly of Connection Port 40>

In assembling the connection port 40 configured as described above, the integrally molded product 660 is inserted into the second hole portion 63c of the medicinal solution intake port portion 63 and, with the O-ring 66 inserted in the first hole portion 63b of the medicinal solution intake port portion 63, the cap portion 67 is attached to the distal portion of the medicinal solution intake port portion 63. By locking the locking claw 67b of the cap portion 67 to the stepped portion 63a of the medicinal solution intake port portion 63 at this time, the cap portion 67 is prevented from coming off from the medicinal solution intake port portion 63. In addition, in the medicinal solution intake port portion 63, a gap is formed between the outer peripheral surface of the integrally molded product 660 and the inner peripheral surface of the second hole portion 63c. This is because the diameter of the integrally molded product 660 is set to be smaller than the diameter of the second hole portion 63c of the medicinal solution intake port portion 63. In addition, in the medicinal solution intake port portion 63, one end of the biasing unit 640 in the X1 direction is locked into the medicinal solution intake port portion 63 and the plug portion 650 is pressed against the liquid introduction hole 67a side of the cap portion 67, that is, the O-ring 66 by the biasing force of the biasing unit 640. As a result, the liquid introduction hole 67a of the cap portion 67 is blocked by the plug portion 650. In addition, the plug portion 650 comes into close contact with the O-ring 66 and the O-ring 66 comes into close contact with the inner peripheral surface of the first hole portion 63b. As a result, the distal portion of the medicinal solution intake port portion 63 is liquid-tightly sealed. The biasing unit 640, the O-ring 66, and the plug portion 650 configure a non-puncture-type sealing mechanism in the second connection portion 620.

Next, a case where the device main body 20 is mounted on the cradle 30 and a case where the device main body 20 is removed from the cradle 30 in the medicinal solution administration device according to the second embodiment of the invention will be described.

### <Case Where Device Main Body 20 Is Mounted on Cradle 30>

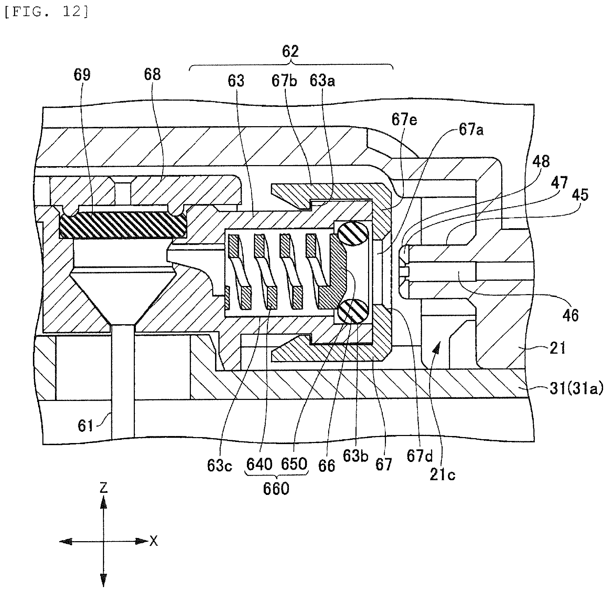

In a case where the device main body 20 is mounted on the cradle 30 in using the medicinal solution administration device 1, the device main body 20 is slid in the X1 direction with respect to the cradle 30 as described with reference to Fig. 8. Then, the distal portion of the liquid delivery port portion 45 is disposed in the vicinity of the cap portion 67 as illustrated in Fig. 12. Next, the distal portion of the liquid delivery port portion 45 is inserted into the liquid introduction hole 67a of the cap portion 67. At this time, the first tapered guide 48 and the second tapered guide 67d engage with each other to guide the distal portion of the liquid delivery port portion 45 to the liquid introduction hole 67a of the cap portion 67. Next, the distal portion of the liquid delivery port portion 45 comes into contact with the plug portion 650 through the liquid introduction hole 67a of the cap portion 67 and the hole of the O-ring 66. At this time, a space is formed between the outside surface of the plug portion 650 (outer surface on the X2 direction side, that is, surface exposed to the liquid introduction hole 67a) and the distal surface of the liquid delivery port portion 45 by the groove 47 formed in the distal portion of the liquid delivery port portion 45. Accordingly, a gap corresponding to the recess dimension of the groove 47 is formed between the groove 47 of the liquid delivery port portion 45 and the distal surface of the plug portion 650.

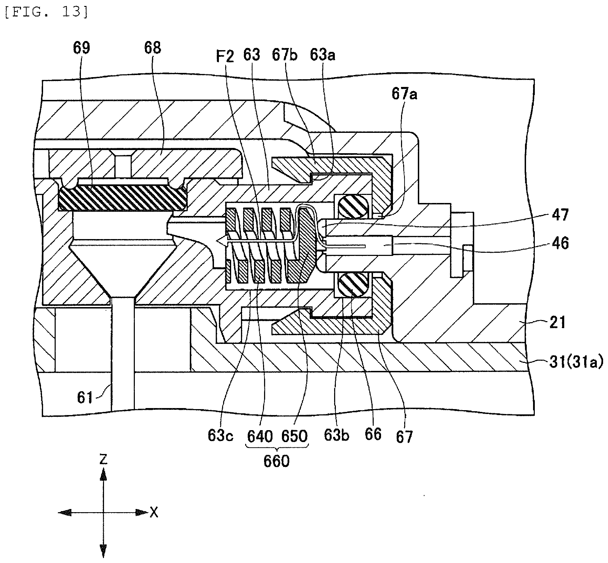

Next, the liquid delivery port portion 45 pushes the plug portion 650 into the back side of the medicinal solution intake port portion 63 against the biasing force of the biasing unit 640. As a result, the plug portion 650 moves away from the O-ring 66 and moves to the back side of the medicinal solution intake port portion 63. Then, with the device main body 20 completely mounted on the cradle 30, the liquid delivery port portion 45 is in the cap portion 67 as illustrated in Fig. 13. In this state, a medicinal solution flow path is formed by the plurality of grooves 47 at the contact part between the distal portion of the liquid delivery port portion 45 and the plug portion 650. Accordingly, the liquid delivery hole 46 of the liquid delivery port portion 45 communicates with the first hole portion 63b and the second hole portion 63c of the medicinal solution intake port portion 63 through the grooves 47.

As a result, the first connection portion 21c and the second connection portion 620 are connected and a medicinal solution flow path F2 as indicated by an arrow is formed at the connection part. This medicinal solution flow path F2 is a flow path connected to the cannula 61 in the housing 68. Accordingly, the liquid delivery tube 24 connected to the medicinal solution storage portion 22 and the cannula 61 piercing a living body are connected by the first connection portion 21c and the second connection portion 62. Accordingly, by sending out the medicinal solution stored in the outer tube 22a of the medicinal solution storage portion 22 to the liquid delivery tube 24 with the plunger 22b, the medicinal solution is capable of flowing into the medicinal solution intake port portion 63 through the liquid delivery hole 46 and the groove 47 of the liquid delivery port portion 45. Then, the medicinal solution that has flowed into the medicinal solution intake port portion 63 is capable of flowing into the cannula 61 through the internal space of the housing 68 and can be introduced into a living body from the distal end of the cannula 61.

### <Case Where Device Main Body 20 Is Removed from Cradle 30>

In a case where the device main body 20 is removed from the cradle 30 for some reason during the use of the medicinal solution administration device 1, the device main body 20 is slid in the X2 direction (see Fig. 8), which is opposite to the mounting direction. As a result, the device main body 20 slides from the second end 30b toward the first end 30a of the cradle 30 and the first connection portion 21c and the second connection portion 62 are disconnected as a result of this sliding. The following is a detailed description.

When the device main body 20 is slid in the X2 direction with respect to the cradle 30, first, the liquid delivery port portion 45 moves in the X2 direction together with the device main body 20 and the plug portion 650 also moves in the X2 direction as the liquid delivery port portion 45 moves. At this time, the plug portion 650 is pushed by the biasing force of the biasing unit 640 and moves in the X2 direction. Next, the plug portion 650 comes into contact with the O-ring 66 by the liquid delivery port portion 45 moving further. As a result, the plug portion 650 is pressed against the O-ring 66 by the biasing force of the biasing unit 640, and thus the distal portion of the medicinal solution intake port portion 63 is self-sealed. Next, the distal portion of the liquid delivery port portion 45 escapes to the outside of the cap portion 67 through the liquid introduction hole 67a of the cap portion 67. As a result, the first connection portion 21c and the second connection portion 62 are disconnected.

### <Effect of Second Embodiment>

As described above, in the medicinal solution administration device 1 according to the second embodiment of the invention, the same effect as that of the first embodiment can be obtained. In the second embodiment of the invention, the liquid delivery port portion 45 provided in the first connection portion 21c pushes the plug portion 650 into the back side of the medicinal solution intake port portion 63 against the biasing force of the biasing unit 640 in a case where the device main body 20 is mounted on the cradle 30. As a result, a medicinal solution is capable of flowing into the medicinal solution intake port portion 63 from the liquid delivery tube 24 through the liquid delivery hole 46. In addition, in a case where the device main body 20 is removed from the cradle 30, the distal portion of the medicinal solution intake port portion 63 can be self-sealed by pressing the plug portion 650 against the O-ring 66 with the biasing force of the biasing unit 640. In other words, the housing 68 can be returned to the liquid-tightly sealed state and contamination in the medicinal solution intake port 63 can be prevented. As a result, it is possible to suppress medicinal solution leakage attributable to repeated attachment and detachment between the device main body 20 and the cradle 30.

In addition, in the second embodiment of the invention, the biasing unit 640 and the plug portion 650 are an integrated structure, and thus the number of components inserted into the medicinal solution intake port portion 63 is smaller than in a case where the biasing unit 640 and the plug portion 650 are configured by separate components. Accordingly, the assembly man-hours of the connection port 40 can be reduced. In addition, in the second embodiment of the invention, the biasing unit 640 and the plug portion 650 are configured by the integrally molded product 660, and thus the number of components of the connection port 40 can be reduced as compared with a case where the biasing unit 640 and the plug portion 650 are configured by separate components.

An example in which the groove 47 is formed in the distal portion of the liquid delivery port portion 45 has been described in the second embodiment described above, and yet the invention is not limited thereto and one or more grooves may be formed in the distal surface of the plug portion 650. In addition, one or more grooves may be formed in both the distal portion of the liquid delivery port portion 45 and the distal surface of the plug portion 650.

In the second embodiment, the O-ring 66 as a seal portion and the cap portion 67 are configured as separate components, and yet the invention is not limited thereto and the seal portion and the cap portion 67 may be an integrated structure. Specifically, the seal portion and the cap portion 67 may be configured by a two-color molded product. By the seal portion and the cap portion 67 being an integrated structure, the number of components of the connection port 40 can be reduced. In addition, by the seal portion and the cap portion 67 being configured by a two-color molded product, it is possible to save the trouble of attaching the seal portion to the medicinal solution intake port portion 63. Accordingly, the assembly man-hours of the connection port 40 can be reduced. The point that the seal portion and the cap portion 67 are integrated and the point that the seal portion and the cap portion 67 are configured by a two-color molded product are similarly applicable to the first embodiment.

In the second embodiment, the O-ring 66 is used as a seal portion and the distal portion of the medicinal solution intake port portion 63 is blocked by pressing the plug portion 650 against the O-ring 66, and yet the invention is not limited thereto. In an alternative configuration, the cap portion 67 may be used as a seal portion and the distal portion of the medicinal solution intake port portion 63 may be blocked by pressing the plug portion 650 against the liquid introduction hole 67a of the cap portion 67. In this configuration, the O-ring 66 is unnecessary, and thus the number of components of the connection port 40 can be reduced and the assembly man-hours of the connection port 40 can be reduced.

### [Third Embodiment]

A third embodiment of the invention will be described below. The medicinal solution administration device 1 according to the third embodiment of the invention is different from the device configuration of the second embodiment described above in terms of the configurations of the first connection portion 21c and the connection port 40. The following is a detailed description.

### (First Connection Portion 21c)

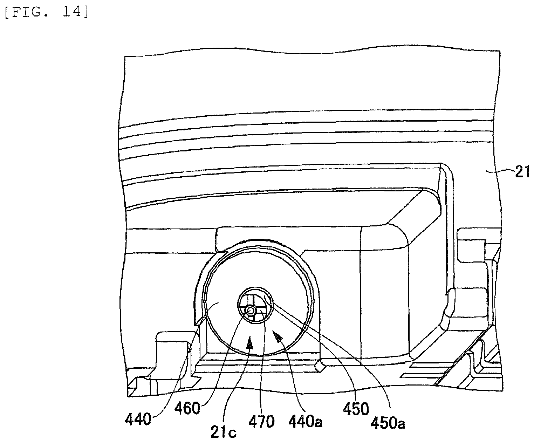

Fig. 14 is a perspective view illustrating a main part of the device main body according to the third embodiment of the invention. As illustrated in Fig. 14, the housing 21 of the device main body 20 is provided with the first connection portion 21c. The first connection portion 21c is provided with a concave portion 440, a concave liquid delivery port portion 450, and a liquid delivery hole 460. The concave portion 440 is formed in a circular ring shape when viewed from the X direction and is concave in a substantially U shape in the X direction. The liquid delivery port portion 450 is open to a bottom surface 440a of the concave portion 440. The liquid delivery port portion 450 is provided at the center of the concave portion 440 and is formed in a circular shape when viewed from the X direction. A third tapered guide 450a is formed on the opening edge of the liquid delivery port portion 450. The third tapered guide 450a is formed so as to chamfer the opening edge of the liquid delivery port portion 450. In addition, the liquid delivery port portion 450 is concave in a substantially U shape in the X direction from the bottom surface 440a of the concave portion 440. The bottom surface 440a of the concave portion 440 faces a medicinal solution intake port portion 630 of the connection port 40 in a case where the device main body 20 is mounted on the cradle 30.

The liquid delivery hole 460 is open on the back side (X1 direction side) surface of the liquid delivery port portion 450. The back side surface of the liquid delivery port portion 450 is positioned on the side opposite to the third tapered guide 450a in the X direction. A plurality of grooves 470 are formed, together with the liquid delivery hole 460, in the back side surface of the liquid delivery port portion 450. Four grooves 470 are formed in the present embodiment. The four grooves 470 are formed radially (in a cross shape) around the liquid delivery hole 460. An end portion of the liquid delivery tube 24 (see Fig. 2) is connected to the liquid delivery hole 460.

### (Connection Port 40)

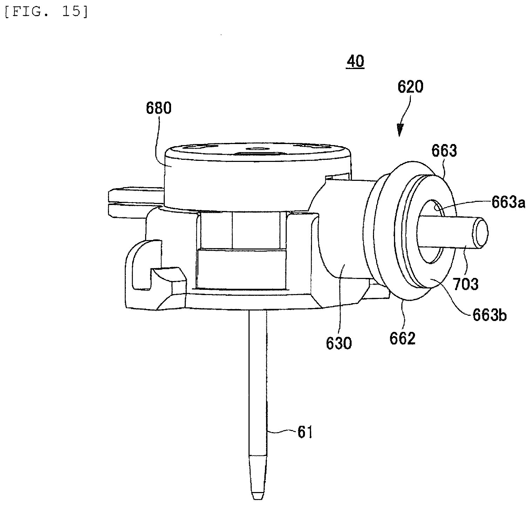

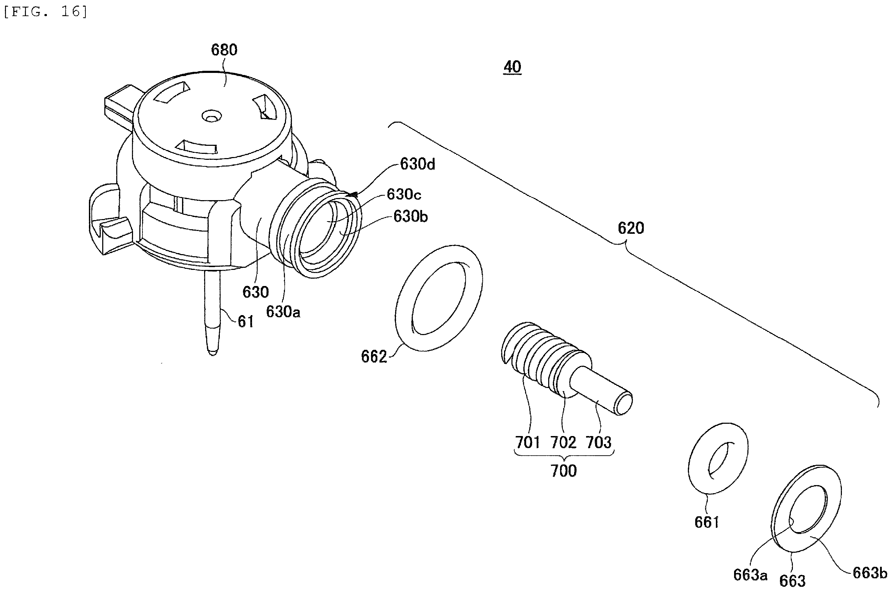

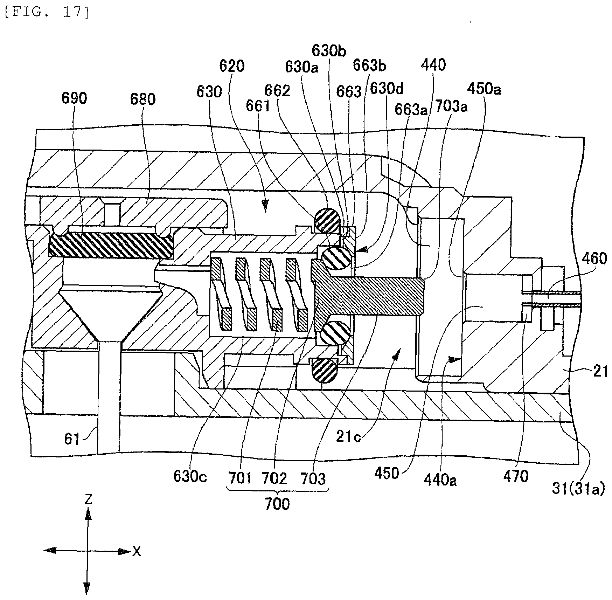

Fig. 15 is a perspective view of the connection port 40 of the medicinal solution administration device 1 according to the third embodiment of the invention, and Fig. 16 is an exploded perspective view of the connection port 40 of the medicinal solution administration device 1 according to the third embodiment of the invention. As illustrated in Figs. 15 and 16, the connection port 40 includes the cannula 61, a housing 680, a rubber stopper 690 (see Fig. 17), and the second connection portion 620. The configurations of the cannula 61, the housing 680, the rubber stopper 690, the puncture device (not illustrated), and the inner needle (not illustrated) are the same as those of the first embodiment. The second connection portion 620 includes the medicinal solution intake port portion 630, a first O-ring 661, a second O-ring 662, an integrally molded product 700, and a cap portion 663.

The medicinal solution intake port portion 630 is formed in a cylindrical shape. A peripheral groove 630a is formed on the outer peripheral side of the medicinal solution intake port portion 630. The peripheral groove 630a is a groove for attaching the second O-ring 662 on the outer peripheral side of the medicinal solution intake port portion 630. A first hole portion 630b and a second hole portion 630c are formed on the inner peripheral side of the medicinal solution intake port portion 630. The diameter of the first hole portion 630b is set to be larger than the diameter of the second hole portion 630c. The second hole portion 630c communicates with the cannula 61 in the housing 680. The medicinal solution intake port portion 630 is formed integrally with the housing 680. The housing 680 configures the main body portion of the connection port 40. The cannula 61 described above is attached to the housing 680.

The first O-ring 661 corresponds to a seal portion. The first O-ring 661 is attached to the first hole portion 630b of the medicinal solution intake port portion 630. The outer peripheral surface of the first O-ring 661 is in close contact with the inner peripheral surface of the first hole portion 630b. The second O-ring 662 corresponds to a second seal portion. The second O-ring 662 is attached to the peripheral groove 630a of the medicinal solution intake port portion 630. The inner peripheral surface of the second O-ring 662 is in close contact with the outer peripheral surface of the peripheral groove 630a. The diameter of the outer peripheral surface of the second O-ring 662 is set to be larger by a predetermined amount than the diameter of the inner peripheral surface of the concave portion 440. The "predetermined amount" described here is an amount at which the second O-ring 662 can be inserted into the concave portion 440 as a result of the elastic deformation of the second O-ring 662 (crushing of cross-sectional shape).

The cap portion 663 is attached to the distal portion of the medicinal solution intake port portion 630. The distal portion of the medicinal solution intake port portion 630 is the end portion on the side facing the first connection portion 21c (see Fig. 3) when the device main body 20 is mounted on the cradle 30. The cap portion 663 has a liquid introduction hole 663a.

The liquid introduction hole 663a is formed at a front plate part 663b of the cap portion 663 and is formed in a circular shape when the cap portion 663 is viewed from the X direction. The liquid introduction hole 663a is a through hole that leads to the inner portion of the medicinal solution intake port portion 630 and where a nose portion 703 protrudes in a case where the cap portion 663 is attached to the medicinal solution intake port portion 630. The liquid introduction hole 663a is provided at the center of the cap portion 663 when viewed from the X direction.

The integrally molded product 700 is made of resin and has a biasing unit 701, a plug portion 702, and the nose portion 703 integrally. Examples of the resin configuring the integrally molded product 700 include engineering plastics such as polyacetal resin and ABS resin. The biasing unit 701 is formed in a spiral shape and has an appropriate springiness. The plug portion 702 is formed in a disk shape. The nose portion 703 is formed in a columnar shape. The nose portion 703 is disposed so as to protrude from the distal portion of the medicinal solution intake port portion 630. The distal portion of the nose portion 703 is provided with a fourth tapered guide 703a. The fourth tapered guide 703a is formed so as to correspond to the third tapered guide 450a described above. The fourth tapered guide 703a is formed so as to chamfer the distal portion of the nose portion 703. The diameter of the nose portion 703 is set to be smaller than the inner diameter of the liquid delivery port portion 450. In addition, the protrusion dimension of the nose portion 703 with respect to a distal surface 630d of the medicinal solution intake port portion 630 is set to be larger than the depth dimension of the liquid delivery port portion 450 with respect to the bottom surface 440a of the concave portion 440.

### <Assembly of Connection Port 40>

In assembling the connection port 40 configured as described above, the biasing unit 701 and the plug portion 702 of the integrally molded product 700 are inserted into the second hole portion 630c of the medicinal solution intake port portion 630, the first O-ring 661 is inserted into the first hole portion 63b of the medicinal solution intake port portion 630, and the cap portion 663 is attached to the distal portion of the medicinal solution intake port portion 630. By passing the nose portion 703 of the integrally molded product 700 through the hole of the first O-ring 661 and the liquid introduction hole 663a of the cap portion 663 at this time, the nose portion 703 is caused to protrude from the distal portion of the medicinal solution intake port portion 630. In addition, the second O-ring 662 is attached to the peripheral groove 630a of the medicinal solution intake port portion 630. At this time, a gap is formed between the outer peripheral surface of the biasing unit 701 and the inner peripheral surface of the second hole portion 630c in the medicinal solution intake port portion 630. This is because that the diameters of the biasing unit 701 and the plug portion 702 in the integrally molded product 700 are set to be smaller than the diameter of the second hole portion 630c. In addition, in the medicinal solution intake port portion 630, one end of the biasing unit 701 in the X1 direction is locked into the medicinal solution intake port portion 630 and the plug portion 702 is pressed against the first O-ring 661 by the biasing force of the biasing unit 701. As a result, the plug portion 702 comes into close contact with the first O-ring 661, and thus the distal portion of the medicinal solution intake port portion 630 is liquid-tightly sealed.

Next, a case where the device main body 20 is mounted on the cradle 30 and a case where the device main body 20 is removed from the cradle 30 in the medicinal solution administration device according to the third embodiment of the invention will be described.

### <Case Where Device Main Body 20 Is Mounted on Cradle 30>

In a case where the device main body 20 is mounted on the cradle 30 in using the medicinal solution administration device 1, the device main body 20 is slid in the X1 direction with respect to the cradle 30 as described with reference to Fig. 8. As a result, the first connection portion 21c is disposed in the vicinity of the nose portion 703 as illustrated in Fig. 17. Next, the nose portion 703 is inserted into the liquid delivery port portion 450 through the concave portion 440. At this time, the distal portion of the nose portion 703 is guided into the liquid delivery port portion 450 by the third tapered guide 450a and the fourth tapered guide 703a. Accordingly, the nose portion 703 can be smoothly inserted with respect to the liquid delivery port portion 450. By the nose portion 703 being inserted into the liquid delivery port portion 450, a gap is formed between the outer peripheral surface of the nose portion 703 and the inner peripheral surface of the liquid delivery port portion 450. This is because that the diameter of the nose portion 703 in the integrally molded product 700 (outer diameter) is set to be smaller than the diameter of the liquid delivery port portion 450 (inner diameter).

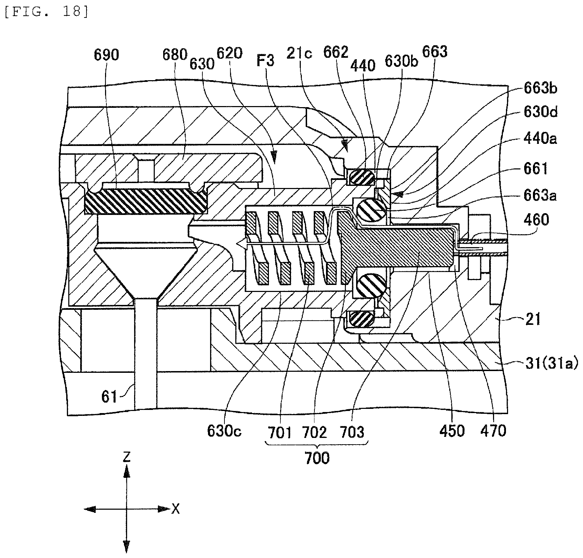

Next, the distal portion of the medicinal solution intake port portion 630 is inserted into the concave portion 440 and the second O-ring 662 is inserted into the concave portion 440 while being elastically deformed. As a result, the outer peripheral surface of the second O-ring 662 comes into close contact with the inner peripheral surface of the concave portion 440. The back side surface of the liquid delivery port portion 450 comes into contact with the distal portion of the nose portion 703 and, in that state, pushes the nose portion 703 into the second hole portion 630c side against the biasing force of the biasing unit 701. As a result, the plug portion 702 moves away from the first O-ring 661 and moves to the back side of the medicinal solution intake port portion 630. In other words, the nose portion 703 and the plug portion 702 are displaced from the initial positions to the X1 direction in the medicinal solution intake port portion 630. Then, with the device main body 20 completely mounted on the cradle 30, the bottom surface 440a of the concave portion 440 is close to the distal portion of the medicinal solution intake port portion 630 as illustrated in Fig. 18. In this state, a medicinal solution flow path is formed by the plurality of grooves 470 at the contact part between the back side surface of the liquid delivery port portion 450 and the distal surface of the nose portion 703. Accordingly, the liquid delivery hole 460 of the first connection portion 21c communicates with the first hole portion 630b and the second hole portion 630c of the medicinal solution intake port portion 630 through the grooves 470 and the gap between the nose portion 703 and the liquid delivery port portion 450.

As a result, the first connection portion 21c and the second connection portion 620 are connected and a medicinal solution flow path F3 as indicated by an arrow is formed at the connection part. This medicinal solution flow path F3 is a flow path connected to the cannula 61 in the housing 680. Accordingly, the liquid delivery tube 24 connected to the medicinal solution storage portion 22 and the cannula 61 piercing a living body are connected by the first connection portion 21c and the second connection portion 620. Accordingly, by sending out the medicinal solution stored in the outer tube 22a of the medicinal solution storage portion 22 to the liquid delivery tube 24 with the plunger 22b, the medicinal solution is capable of flowing into the medicinal solution intake port portion 630 through the liquid delivery hole 460 and the plurality of grooves 470 of the first connection portion 21c and, further, through the gap between the nose portion 703 and the liquid delivery port portion 450. Then, the medicinal solution that has flowed into the medicinal solution intake port portion 630 is capable of flowing into the cannula 61 through the internal space of the housing 680 and can be introduced into a living body from the distal end of the cannula 61.

### <Case Where Device Main Body 20 Is Removed from Cradle 30>

In a case where the device main body 20 is removed from the cradle 30 for some reason during the use of the medicinal solution administration device 1, the device main body 20 is slid in the X2 direction (see Fig. 8), which is opposite to the mounting direction. As a result, the device main body 20 slides from the second end 30b toward the first end 30a of the cradle 30 and the first connection portion 21c and the second connection portion 620 are disconnected as a result of this sliding. The following is a detailed description.

When the device main body 20 is slid in the X2 direction with respect to the cradle 30, first, the liquid delivery port portion 450 of the first connection portion 21c moves in the X2 direction together with the device main body 20 and the nose portion 703 and the plug portion 702 also move in the X2 direction as the liquid delivery port portion 450 moves. At this time, the nose portion 703 and the plug portion 702 are pushed by the biasing force of the biasing unit 701 and move in the X2 direction (return to the initial positions). Next, the plug portion 702 comes into contact with the first O-ring 661 by the liquid delivery port portion 450 moving further and the plug portion 702 is pressed against the first O-ring 661 by the biasing force of the biasing unit 701. As a result, the distal portion of the medicinal solution intake port portion 630 is blocked by the plug portion 702. As a result, the distal portion of the medicinal solution intake port portion 630 returns to the liquid-tightly sealed state. Next, the nose portion 703 escapes from the liquid delivery port portion 450 after the back side surface of the liquid delivery port portion 450 moves away from the distal portion of the nose portion 703. As a result, the first connection portion 21c and the second connection portion 620 are disconnected.

### <Effect of Third Embodiment>

As described above, in the medicinal solution administration device 1 according to the third embodiment of the invention, the same effect as those of the first and second embodiments can be obtained. In a case where the device main body 20 is mounted on the cradle 30 in the third embodiment of the invention, the back side surface of the liquid delivery port portion 450 in the first connection portion 21c comes into contact with the nose portion 703 and, in this contact state, the back side surface of the liquid delivery port portion 450 pushes the nose portion 703 and the plug portion 702 into the back side of the medicinal solution intake port portion 630 against the biasing force of the biasing unit 701. As a result, a medicinal solution is capable of flowing into the medicinal solution intake port portion 63 from the liquid delivery tube 24 through the liquid delivery hole 460. In addition, in a case where the device main body 20 is removed from the cradle 30, the distal portion of the medicinal solution intake port portion 630 can be sealed by pressing the plug portion 702 against the first O-ring 661 with the biasing force of the biasing unit 701. In other words, the housing 680 can be self-sealed. As a result, it is possible to suppress medicinal solution leakage attributable to repeated attachment and detachment between the device main body 20 and the cradle 30. Since a needle member and a diaphragm are not used for the fluid connection between the device main body 20 and the cradle 30, it is possible to suppress diaphragm damage and coring attributable to repeated attachment and detachment between the device main body 20 and the cradle 30.

In addition, in the third embodiment of the invention, the first O-ring 661 is provided in the medicinal solution intake port portion 630 and the plug portion 702 is pressed against the first O-ring 661 by the biasing force of the biasing unit 701. Accordingly, the distal portion of the medicinal solution intake port portion 630 can be blocked with high liquid tightness.

In addition, in the third embodiment of the invention, the second O-ring 662 is provided on the outer peripheral side of the medicinal solution intake port portion 630 and the outer peripheral surface of the second O-ring 662 is brought into close contact with the inner peripheral surface of the concave portion 440. Accordingly, it is possible to suppress liquid leakage from the fitting part between the medicinal solution intake port portion 630 and the concave portion 440.

The plurality of grooves 470 are formed in the back side surface of the liquid delivery port portion 450 in the third embodiment, and yet the invention is not limited thereto. In alternative configurations, one, two, three, or five or more grooves 470 may be formed in the back side surface of the liquid delivery port portion 450. In a case where the plurality of grooves 470 are formed in the back side surface of the liquid delivery port portion 450, the flow path resistance of the medicinal solution flow path at the contact part between the back side surface of the liquid delivery port portion 450 and the nose portion 703 is smaller than in a case where only one groove 470 is formed. Accordingly, a medicinal solution can be smoothly delivered from the liquid delivery port portion 450 to the medicinal solution intake port portion 630. In addition, in a case where the plurality of grooves 470 are formed in the back side surface of the liquid delivery port portion 450, the plurality of grooves 470 are radially formed around the liquid delivery hole 460. As a result, a medicinal solution can be more smoothly delivered from the liquid delivery port portion 450 to the medicinal solution intake port portion 630 and the medicinal solution can be more uniformly delivered in the circumferential direction of the nose portion 703.

In the third embodiment, the first O-ring 661 as a seal portion and the medicinal solution intake port portion 630 are configured as separate components, and yet the invention is not limited thereto and the seal portion and the medicinal solution intake port portion 630 may be an integrated structure. Specifically, the seal portion and the medicinal solution intake port portion 630 may be configured by a two-color molded product. By the seal portion and the medicinal solution intake port portion 630 being an integrated structure, the number of components of the connection port 40 can be reduced. In addition, by the seal portion and the medicinal solution intake port portion 630 being configured by a two-color molded product, it is possible to save the trouble of attaching the seal portion to the medicinal solution intake port portion 630. Accordingly, the assembly man-hours of the connection port 40 can be reduced.

In the third embodiment, the second O-ring 662 as a second seal portion and the medicinal solution intake port portion 630 are configured as separate components, and yet the invention is not limited thereto and the second seal portion and the medicinal solution intake port portion 630 may be an integrated structure. Specifically, the second seal portion and the medicinal solution intake port portion 630 may be configured by a two-color molded product. By the second seal portion and the medicinal solution intake port portion 630 being an integrated structure, the number of components of the connection port 40 can be reduced. In addition, by the second seal portion and the medicinal solution intake port portion 630 being configured by a two-color molded product, it is possible to save the trouble of attaching the second seal portion to the medicinal solution intake port portion 630. Accordingly, the assembly man-hours of the connection port 40 can be reduced.

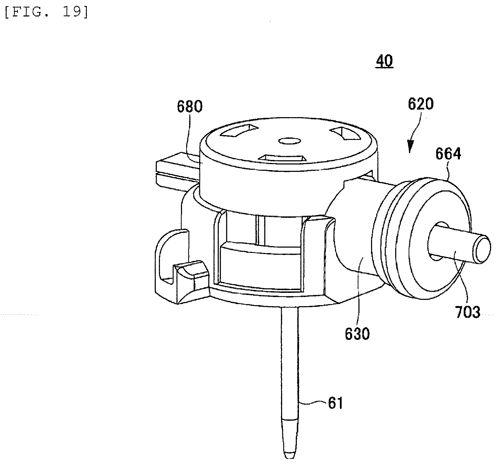

In the third embodiment, the first O-ring 661 as a first seal portion, the second O-ring 662 as a second seal portion, and the cap portion 663 are configured as separate components, and yet the invention is not limited thereto and the first seal portion, the second seal portion, and the cap portion 663 may be an integrated structure. By the first seal portion, the second seal portion, and the cap portion 663 being an integrated structure, the number of components of the connection port 40 can be reduced. Specifically, by the first seal portion, the second seal portion, and the cap portion being configured as an integrally molded product 664 made of an elastic member as illustrated in Fig. 19, it is possible to save the trouble of attaching each component and enhance the liquid tightness between the liquid delivery port portion 450 and the medicinal solution intake port portion 630.

An example in which the groove 470 is formed in the back side surface of the liquid delivery port portion 450 has been described in the third embodiment described above, and yet the invention is not limited thereto and one or more grooves may be formed in the distal surface of the nose portion 703. In addition, one or more grooves may be formed in both the back side surface of the liquid delivery port portion 450 and the distal surface of the nose portion 703.

### Reference Signs List

1: Medicinal solution administration device, 20: Device main body, 21c: First connection portion, 22: Medicinal solution storage portion, 24: Liquid delivery tube, 30: Cradle, 40: Connection port, 45: Liquid delivery port portion, 46: Liquid delivery hole, 47: Groove, 48: First tapered guide, 62: Second connection portion, 63: Medicinal solution intake port portion, 64: Spring (elastic member, biasing unit), 65: Steel ball (plug portion), 66: O-ring (seal portion), 67: Cap portion, 67a: Liquid introduction hole, 67d: Second tapered guide, 450: Liquid delivery port portion, 450a: Third tapered guide, 460: Liquid delivery hole, 470: Groove, 640: Biasing unit, 650: Plug portion, 660: Integrally molded product, 661: First O-ring (seal portion), 662: Second O-ring (second seal portion), 700: Integrally molded product, 701: Biasing unit, 702: Plug portion, 703: Nose portion, 703a: Fourth tapered guide, F1, F2, F3: Medicinal solution flow path

## Claims

1. A medicinal solution administration device comprising a device main body and a cradle detachable from each other, wherein
in the device main body, a liquid delivery tube extending from a medicinal solution storage portion is provided and a first connection portion where a liquid delivery hole extending from the liquid delivery tube is open is formed,
the cradle is provided with a connection port connected to the liquid delivery tube via the first connection portion,
the connection port has a second connection portion connectable to the first connection portion,
the second connection portion has a medicinal solution intake port portion, a seal portion provided in the medicinal solution intake port portion, a plug portion provided in the medicinal solution intake port portion and movable in the medicinal solution intake port portion, and a biasing unit blocking a distal portion of the medicinal solution intake port portion by pressing the plug portion against the seal portion,
in a case where the device main body is mounted on the cradle, a medicinal solution is capable of flowing into the medicinal solution intake port portion from the liquid delivery tube through the liquid delivery hole by the first connection portion displacing the plug portion in the medicinal solution intake port portion against a biasing force of the biasing unit, and
in a case where the device main body is removed from the cradle, the distal portion of the medicinal solution intake port portion is blocked by the plug portion being biased toward the seal portion by the biasing force of the biasing unit.

2. The medicinal solution administration device according to claim 1, wherein
the first connection portion is provided with a convex liquid delivery port portion having the liquid delivery hole, and
the liquid delivery port portion pushes the plug portion into a back side of the medicinal solution intake port portion against the biasing force of the biasing unit as the device main body is mounted on the cradle.

3. The medicinal solution administration device according to claim 2, wherein at least one of the liquid delivery port portion and the plug portion is provided with a groove forming, in a case where a distal portion of the liquid delivery port portion is in contact with the plug portion, a medicinal solution flow path at the contact part.

4. The medicinal solution administration device according to any one of claims 1 to 3, wherein
the second connection portion further includes a cap portion attached to the distal portion of the medicinal solution intake port portion, and
the cap portion has a medicinal solution introduction hole leading to an inner portion of the medicinal solution intake port portion.

5. The medicinal solution administration device according to claim 4, wherein
the plug portion is made of a steel ball,
the seal portion is positioned between the steel ball and the cap portion, and
the biasing unit biases the steel ball so as to be pressed against the seal portion.

6. The medicinal solution administration device according to claim 4 or 5, wherein
the first connection portion is provided with a convex liquid delivery port portion having the liquid delivery hole,
a first tapered guide is formed on the distal portion of the liquid delivery port portion,
a second tapered guide is formed on an inner peripheral edge of the medicinal solution introduction hole of the cap portion, and
the distal portion of the liquid delivery port portion is guided to the medicinal solution introduction hole of the cap portion by the first tapered guide and the second tapered guide engaging with each other.

7. The medicinal solution administration device according to claim 1, wherein
the second connection portion further has a nose portion formed integrally with the plug portion and protruding from the distal portion of the medicinal solution intake port portion, and
the first connection portion comes into contact with the nose portion and, in the contact state, pushes the plug portion into a back side of the medicinal solution intake port portion against the biasing force of the biasing unit as the device main body is mounted on the cradle.

8. The medicinal solution administration device according to claim 7, wherein
the first connection portion has a concave liquid delivery port portion into which the nose portion is inserted, and
at least one of the nose portion and the liquid delivery port portion is provided with a groove forming, in a case where a back side surface of the liquid delivery port portion is in contact with a distal portion of the nose portion, a medicinal solution flow path at the contact part.

9. The medicinal solution administration device according to claim 8, wherein
the first connection portion has a concave portion where a liquid delivery hole of the liquid delivery port portion is open,
a second seal portion is provided on an outer peripheral side of the medicinal solution intake port portion, and
an outer peripheral surface of the second seal portion is disposed in close contact with an inner peripheral surface of the concave portion.

10. The medicinal solution administration device according to claim 9, wherein
a third tapered guide is formed on an opening edge of the liquid delivery port portion,
a fourth tapered guide is formed on the distal portion of the nose portion, and
the distal portion of the medicinal solution intake port portion is guided into the liquid delivery port portion by the third tapered guide and the fourth tapered guide engaging with each other.

11. The medicinal solution administration device according to any one of claims 1 to 10, wherein the plug portion and the biasing unit are an integrated structure.

12. The medicinal solution administration device according to any one of claims 1 to 11, wherein the seal portion is configured by an O-ring.

13. The medicinal solution administration device according to any one of claims 1 to 12, wherein the medicinal solution intake port portion and the seal portion are an integrated structure.

14. The medicinal solution administration device according to claim 2 or 3, wherein the seal portion and an outer peripheral surface of the liquid delivery port portion are liquid-tightly connected in a state where the device main body and the cradle are operably connected.

15. The medicinal solution administration device according to claim 14, wherein a medicinal solution flow path is formed at a connection part between the first connection portion and the second connection portion in the state where the device main body and the cradle are operably connected.
